# EUROPEAN PATENT SPECIFICATION

(11) **EP 2 667 791 B1**
(45) Date of publication and mention of the grant of the patent: **15.03.2017**
(21) Application number: 12739063.1
(22) Date of filing: 25.01.2012
(51) Int. Cl.: A61B 17/04, A61B 17/06, A61L 27/56

(54) **BARBS FOR FIXATION OF BIOLOGIC PLASTICS**
WIDERHAKEN ZUR FIXIERUNG BIOLOGISCHER KUNSTSTOFFE
ARDILLONS POUR FIXATION DE MATIÈRES PLASTIQUES BIOLOGIQUES

(30) Priority: 26.01.2011 US 201161436412 P
(43) Date of publication of application: 04.12.2013
(73) Proprietor: Carmell Therapeutics Corporation, Pittsburgh, PA 15212 (US)
(72) Inventor: CAMPBELL, Phil G., Cranberry Township Pennsylvania 16066 (US); SMITH, Jason, Pittsburgh Pennsylvania 15229 (US); WEISS, Lee E., Pittsburgh Pennsylvania 15217 (US); WEST, Alan, Pittsburgh Pennsylvania 15213 (US)
(74) Representative: Grund, Martin
(86) International application number: PCT/US2012/022534
(87) International publication number: WO 2012/103205

(56) References cited:
- US-A1- 2002 022 861
- US-A1- 2007 191 761
- US-A1- 2008 286 329
- US-A1- 2008 286 329
- US-A1- 2009 210 006
- FILIZ ET AL: "Micromilling of microbarbs for medical implants", INTERNATIONAL JOURNAL OF MACHINE TOOL DESIGN AND RESEARCH, PERGAMON PRESS, OXFORD, GB, vol. 48, no. 3-4, 4 January 2008 (2008-01-04), pages 459-472, XP022409766, ISSN: 0020-7357, DOI: 10.1016/J.IJMACHTOOLS.2007.08.020
- FILIZ, SINAN ET AL.: 'Micromilling of Microbars for Medical Implants' INTERNATIONAL JOURNAL OF MACHINE TOOLS & MANUFACTURE vol. 48, 07 September 2007, pages 459 - 472, XP022409766

## Description

### Technical Field

The invention pertains to bioresorbable barbs and spikes designed to affix biologically-active blood plasma-derived plastics adjacent to injured tissue for wound coverings, patient implantation devices, or in other medical applications where such affixation is required.

### Background Information

Platelet-rich Plasma (PRP) spun from a patient's own blood has been used since the mid-1980s as a way to accelerate tissue healing. Chronic skin wounds are particularly difficult to treat, and non-healing ulcers are the most frequent cause for amputation. For example, diabetic foot ulcers (DFU) pose a major world-wide health problem due to the associated complications of recurrence, chronicity and amputations. In 2004 in the U.S. there were approximately 71,000 non-traumatic lower-limb amputations in people with diabetes with a cost per patient ranging from $20,000 to $60,000 per case. There has been a dramatic global increase in the prevalence of diabetes mellitus, particularly type II diabetes, which has a lifetime risk of a diabetic patient developing a foot ulcer approaching 25%.
US 2008/286329 A1 discloses blood plasma-derived plastic articles for use as wound repair or tissue graft. The plastic articles can have the form of barbs. US 2009/210006 A1 discloses a compound barb medical device including an elongated body that carries integrally molded barbs. Filiz et al. (International Journal of Machine Tools 2008, 48:459-472) disclose a mechanical micromilling process for the fabrication of microbarbs on medical devices made of biocompatible materials.

### SUMMARY

The invention is defined by the appended claims.

Described herein are barbs or spikes manufactured from blood plasma-derived plastics for fixating tissue with a biologically compatible material that encourages tissue healing. The barbs are formed on blood plasma-derived plastic articles such that the barbs affix the article to injured or repaired tissues. In some embodiments, the barbs are positioned and spaced on a blood plasma-derived plastic such that the barbs are optimally situated. For example, the barbs can be positioned across one face of the scaffold. In some embodiments, the barbs can be positioned along one edge on one face of the scaffold. In some embodiments, the barbs can be positioned around the periphery of one face of the scaffold.

Also provided herein are methods of manufacturing barbs from blood-plasma-derived plastics by molding and post-processing. For example, barbs can be prepared in blood plasma-derived plastic articles such that the plastic barbs have stronger mechanical properties than the blood plasma-derived plastic from which they are constructed. In some embodiments, barbs are prepared using blood plasma-derived plastics such that the barbs controllably swell in place, providing more secure fixation. A means is provided for inserting molding barbed materials into blood plasma-derived plastic articles so as to take advantage of the strength of barbed materials made from different materials than the blood plasma-derived article.

Unless otherwise defined, all technical and scientific terms used herein have the same meaning as commonly understood by one of ordinary skill in the art to which this invention belongs. Although methods and materials similar or equivalent to those described herein can be used to practice the invention, suitable methods and materials are described below. In addition, the materials, methods, and examples are illustrative only and not intended to be limiting.

These and other features, aspects and advantages of the present teachings will become better understood with reference to the following description, examples and appended claims.

### BRIEF DESCRIPTION OF THE DRAWINGS

Figure 1 depicts an SEM photograph of barbs that have been milled in a hard form of fibrin-based plastic, as previously set forth in the International Journal of Machine Tools & Manufacture 48 (2008) 459-472.
Figure 2 shows a method of pressure molding pre-formed molded spikes into barbs.
Figure 3 shows a method of molding a strip of barbs that can later be separated into individual barbs.
Figure 4A shows a method of molding pre-formed barbs into a plastic article. In this case, the barbs can be constructed of a different material than the article. The article into which the barbs are insert-molded can be formed during the molding process. Figure 4B shows the process of compression molding the barbs into the plastic article. Figure 4C shows a flexible scaffold article with insert-molded barbs positioned across one face of the scaffold.
Figure 5A shows positioning of barbs in a scaffold such that the barbs will only contact the periphery of tissue for attachment. In some embodiments this might be useful for a wound covering, for example. Figure 5B shows positioning of barbs at one edge of a scaffold such that in a rolled configuration, the barbs will attach to the scaffold material, providing a tissue wrap. In some embodiments, spacing and positioning is used to tailor the blood plasma-derived apparatus to a specific surgical or medical application.

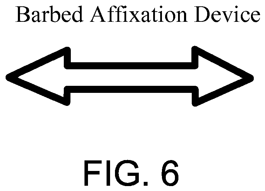

Figure 6 shows a barbed fixation device made from blood plasma-derived plastic such that the device can be used to attach two or more opposing layers of tissue with a device that is biocompatible and encourages the healing of the tissue.

### DETAILED DESCRIPTION

The plastic articles of the present invention are prepared from a composition comprising blood plasma, onto which fixation barbs are insert molded into the articles to affix such articles adjacent to the tissues being treated.

As used herein, the term "plasma" is intended to mean blood plasma, including plasma containing no platelets ("platelet-poor plasma"), plasma containing platelets ("platelet-rich plasma"), or plasma where the platelets have been concentrated by spinning in a centrifuge ("platelet-enriched plasma"). Blood is a bodily fluid (technically a tissue) that is composed of blood cells suspended in a liquid called blood plasma. The blood cells present in blood are red blood cells (also called RBCs or erythrocytes) and white blood cells (including both leukocytes and lymphocytes). Also in blood are platelets (also called thrombocytes), which are cell fragments derived from the fragmentation of precursor megakaryocytes. Plasma makes up about 55% of blood by volume. Blood plasma is essentially an aqueous solution containing about 92% water, about 8% blood plasma proteins (such as serum albumin, blood clotting factors, immunoglobulins (antibodies)), various other proteins, various electrolytes, such as sodium and chloride, and trace amounts of other materials. Blood plasma as used as a component to prepare plastic articles of the present invention refers to the straw-colored liquid portion remaining after the material bodies such as blood cells and cell fragments are separated out from the blood.

Normal platelet counts in human blood average approximately 200,000/µL; PRP has a platelet concentration 3-5 times larger, with a threshold of 1,000,000/µL. PRP also contains multiple natural growth factors and other proteins that appear to accelerate tissue healing. PRP can deliver a concentration of natural growth and regenerative factors to a wound thereby initiating a robust healing cascade that can accelerate the healing of injured tissue. Because PRP can be made from autologous blood, it is relatively safe and free from transmissible diseases from other patients, such as HIV and hepatitis.

Platelets contain numerous growth factors that are released as the platelets are activated. The primary platelet-derived growth factors are: platelet derived growth factor (PDGF-AA, PDGF-BB, PDGF-AB) transforming growth factor beta (TGF-β1, TGF-β2), vascular endothelial growth factor (VEGF), and epithelial growth factor (EGF). These are native growth factors in their biologically correct ratios; this distinguishes PRP from recombinant growth factors. Recombinant growth factors are pure human growth factors, but they are not native growth factors. Instead they are synthesized usually by a culture of Chinese hamster ovarian cells that have a human gene inserted. Recombinant growth factors are single growth factors and are delivered in high doses. PRP is the combination of numerous native growth factors that act by stimulating, for example, cell growth (mitogenesis) and vascularin-growth (angiogenesis).

As used herein, the term "scaffold" is intended to mean either an elastomeric and flexible article that easily contours to the shape of tissue of underlying tissue to which it is positioned, or "scaffold" can mean a rigid plastic article that provides an impetus for encouraging cell growth and healing.

As used herein, the term "barb" is intended to mean a sharp projection with a point design that includes a bent portion (e.g., bent back upon itself, bent forward, bent sideways, or any combination thereof) or with a wedge shape that prevents easy extraction from tissue into which it is inserted. In some embodiments, a barb is less than about 5 mm in length and sufficiently small in diameter (typically less than about 2 mm) such that the insertion of the barb does not cause tissue trauma or undue pain.

As used herein, the term "spike" is intended to mean a sharp pointed projection that can easily be inserted into tissue. In some embodiments, a spike is less than about 5 mm in length and sufficiently small in diameter (typically less than about 2 mm) such that the insertion of the spike does not cause tissue trauma or undue pain.

As used herein, the terms "fixation," "fixate," and "affix" are intended to mean types of physical attachments including attachment with a barb such that the barb cannot be easily removed from the tissue; and attachment with a spike such that the spike prevents shear movement and prevents a spiked apparatus, for example, from sliding across a plane of tissue. Typically, a fixed apparatus containing spikes will not pull away from the tissue by pressure exerted by surrounding tissues.

The source of blood plasma used to prepare the articles of the present invention can include humans and other mammalian species, for example, primates, dogs, cats, rodents and livestock such as sheep, goats, pigs, horses, and cattle. Blood plasma can be pooled plasma from a number of different donors ("allogenic") or can be from an autologous (single) source. As used herein, "allogenic" means that the plasma is taken from different individuals of the same species.

As used herein, "dough" is defined as the combination of plasma powder, plasticizer and any other components that are mixed prior to plasticization.

As used herein, the term "biocompatible" refers to the absence of stimulation of a severe, long-lived or escalating biological response to an implant, and is distinguished from a mild, transient inflammation which typically accompanies surgery or implantation of foreign objects into a living organism.

As used herein, "biological response modifier" means any protein, glycoprotein, polysaccharide, lipid, DNA, RNA, aptamer, peptide, hormone, vitamin and other such substance, which when introduced into a subject is capable of eliciting a biological response, and includes, but is not limited to, cytokines, growth factors, protein hormones, genes, or genetically modified organisms, such as viruses and bacteria, extracellular matrix molecules and the like. The biological response modifier(s) can be present in the blood plasma used to prepare the composition, or added to the composition as a separate component prior to formation of the article.

In some embodiments, the barbs or spikes comprise at least one crosslinking agent for crosslinking various cross-linkable groups of the blood plasma and/or other components of the composition. For example, genipin (Methyl (*1R,2R,6S*)-2-hydroxy-9-(hydroxymethyl)-3-oxabicyclo[4.3.0]nona-4,8-diene-5-carboxylate) can be used as a chemical cross-linker. The amount of crosslinking agent used in the composition can range from about 0.0 to about 0.25 weight percent, or about 0.0 to about 0.5 weight percent, or about 0.0 to about 1.0 weight percent, on a basis of total weight of the composition.

In some embodiments, the composition comprises at least one drug. The term "drug" refers to a substance used as a medication or in the preparation of a medication, including, but not limited to, a substance intended for use in the diagnosis, cure, mitigation, treatment, or prevention of disease. For example, a drug may include, but is not limited to, small organic molecules, complex organic molecules, inorganic elements and molecules, and the like. As used herein, the term "drug" encompasses, for example, fungicides, anticoagulants, antibiotics, antivirals, anti-inflammatories, both steroidal and non-steroidal.

In some embodiments, the articles of the present invention are in the form of stacked or laminated layers of sheets prepared from a blood plasma-derived plastic. Thermal processing of blood-plasma derived plastics imparts additional mechanical strength to the material at the expense of destroying biological factors inherent within the processed blood plasma that direct cell proliferation, differentiation, migration, and function in the process of wound healing. By laminating blood plasma-derived plastics made at different temperatures, a scaffold product that contains a high proportion of inherent biological factors along with greater strength and/or barrier properties can be constructed. In some embodiments, the layers are made with greater amounts of cross-linking agents to provide the benefit of materials that degrade at different rates, providing a combination of longer lasting mechanical and physical properties with a longer and controlled release of growth factors. In some embodiments, the barbs or spikes may be formed in a laminated sheet. For example, the barbs or spikes can be formed at a layer with greater physical and mechanical properties.

Also provided herein are blood plasma-derived soft plastic scaffolds which can be used to enhance the healing of wounds, such as ulcerated (e.g., diabetic foot ulcers) or burned skin, tendon and ligament injuries, nerve damage, and muscular injuries.

Blood plasma-derived plastics can be created by lyophilizing blood plasma and creating a dough from the remaining powder by the addition of a plasticizer (e.g., glycerol) and optionally other components. See, for example, U.S. 2008/0286329; U.S. 2008/0111272; U.S. 2010/0254900; WO 2007/126411; and WO 2009/014776. The dough can be either compression molded or extruded into plastic articles. In some embodiments, spikes that are pre-formed into the plastic articles are incorporated during molding, and barbs are then formed in a post processing procedure. In some embodiments, barbs made of a harder and/or stronger biocompatible and bioresorbable material, for example, a formula of blood plasma-derived plastic different from that of the base article or a synthetic plastic such as poly(methyl methacrylate) (PMMA) or poly(lactic acid) (PLA), are insert molded into the article. In some embodiments, spikes are molded into the blood plasma-derived article. In some embodiments, spikes are made of a material capable of swelling as it hydrates, thereby fixating the article by such expansion. Non-limiting examples of materials capable of swelling upon hydration include certain blood-derived plastics and polysaccharides (e.g., cellulose).

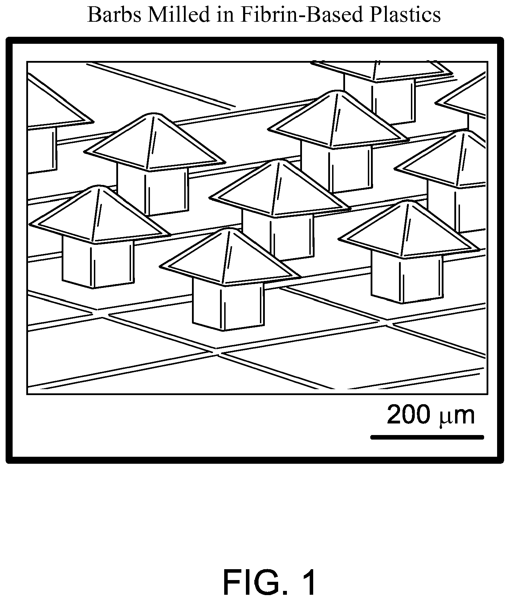

Figure 1 shows machined barbs milled into the surface of a fibrin-based plastic material as discussed in International Journal of Machine Tools & Manufacture 48 (2008) 459-472. The problems with milling such barbs into plastic articles include: 1. the additional processing step required; 2. the cost and time required to perform the milling process; 3. the fact that the length of these barbs is limited to the thickness of the starting material; and 4. the amount of plastic material removed, which can be expensive.

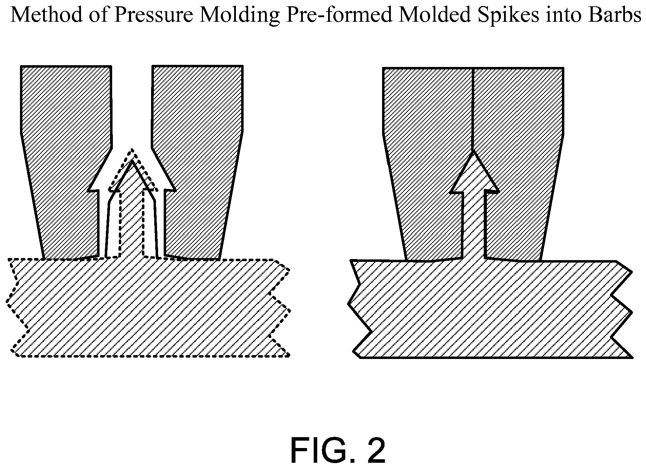

Figure 2 shows a method of post-processing molded spikes to create barbs. In this method, a combination of pressure and temperature is used to mold the individual spikes into a barb shape. Thermal processing of blood-plasma derived plastics imparts additional mechanical strength to the material at the expense of destroying biological factors inherent within the processed blood plasma that direct cell proliferation, differentiation, migration, and function in the process of wound healing. The barbs are designed to temporarily hold the blood plasma-derived plastic articles against injured tissues as the articles degrade, allowing the slow release of growth and regenerative factors that bathe the injured tissues and thereby encourage healing. Therefore, the barbs themselves should be biocompatible but do not need to bioactively participate in the healing process. Accordingly, in some embodiments, the barb molds are heated to relatively high temperatures, typically greater than 100°C. Heating to these temperatures will cause the plastic material of the barbs to strengthen and harden, a benefit for soft and flexible scaffold articles.

Blood plasma-derived plastics can be made to swell as much as 40%. Typically, blood plasma-derived plastics are made using pressures of approximately 10,000 p.s.i. Swelling can be induced by the use of lower pressures, for example, about 3,000 to 10,000 p.s.i. Swelling can also be induced by the use of lower temperatures during processing, for example, temperatures less than about 100°C. In some embodiments, spikes are molded as the article is compression molded by providing cavities into which the material is forced. If these cavities are made so as to be open to their environment, the amount of pressure forming the spikes is appreciably less than the rest of the article, which induces preferential swelling of the spikes over the plastic article.

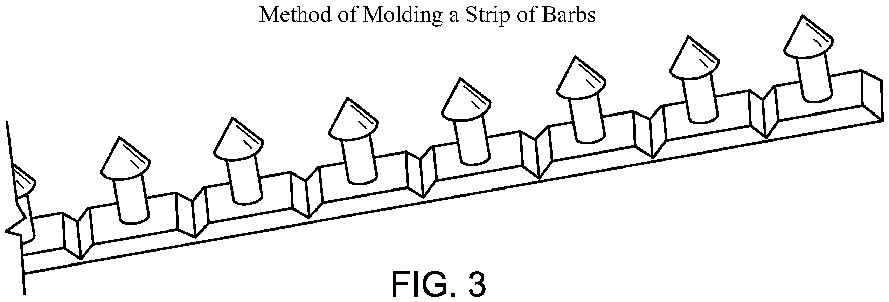

In some embodiments, a tissue attachment spike device incorporates barbs manufactured from blood plasma-derived plastics. These attachment spikes can be manufactured in single or multiple cavity molds. Figure 3 shows a molded strip of such spike devices that can be divided into individual spike devices for attachment to tissue. In some embodiments, the barbed spikes are present on both sides of the device allowing connection or linking of soft tissue to bone or tissue to tissue. Such attachment can encourage healing of the tissues as the articles degrade and release the growth and regenerative factors bound in the plastics.

Also provided herein are barbs made from a material other than a blood plasma-derived plastic. The barbs can be insert-molded into a blood plasma-derived plastic article. For example, a scaffold made from a blood plasma-derived plastic can be contoured around tissue due to its flexibility. In some embodiments, such a scaffold is used to wrap an injured tendon or nerve, or a scaffold might be positioned between a bone and tendon. In some embodiments, the scaffolds are flexible and elastomeric, properties that make it difficult to create effective barbed spikes from the same material as such barbs would be too soft to either penetrate tissue or retain the tissue. In some embodiments, barbs are constructed of a material that is different than the blood plasma-derived plastic scaffold. For example, such barbs can be constructed of different formulations of blood plasma-derived plastics. In some embodiments, the barbs include one or more cross-linking agents or additives, such as tricalcium phosphate (TCP), that provide the plastics with hardness and additional strength. In some embodiments, the barbs are constructed of blood plasma-derived plastics that have been exposed to temperatures exceeding 100°C, thereby creating a blood plasma-derived plastic of greater strength and hardness. In some embodiments, the barbs are constructed of other biocompatible polymers, such as PMMA or PLA.

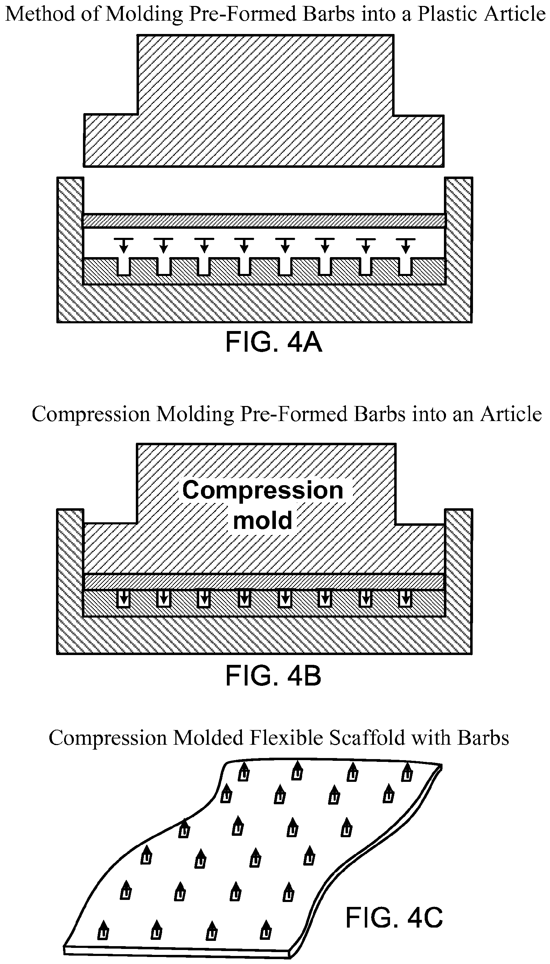

In the present invention, the barbed materials are insert-molded into the scaffold. Figures 4A, 4B, and 4C show one such approach. A dough is made from plasma and placed into the mold into which has been inserted barbed devices. When compression molded, the dough is transformed into a plastic and the barbs locked into the scaffold. For example, in some embodiments barbs could be constructed to enable a flexible scaffold to be wrapped around a tendon or ligament, the barbs holding the scaffold in a tubular shape by penetrating the underlying plastic. In some embodiments, the scaffold is positioned as a wound covering, with the barbs affixing the scaffold device against the dermis.

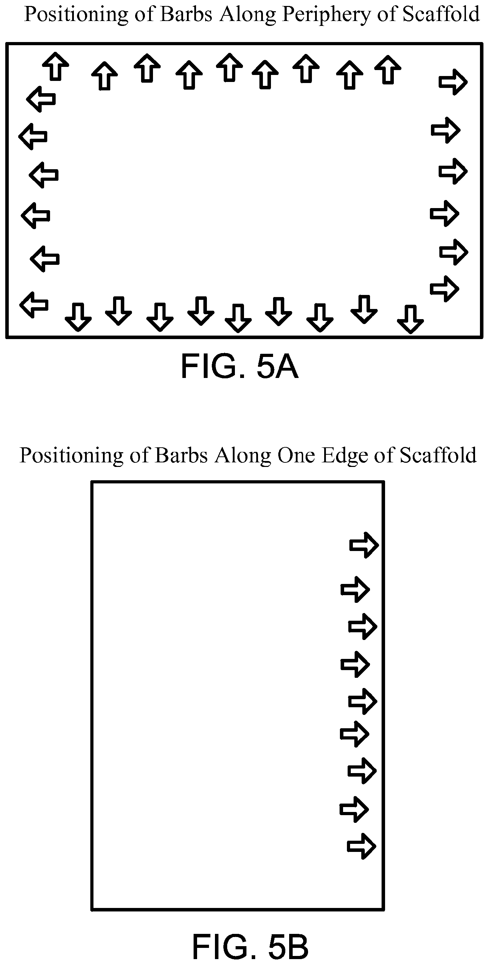

The barbs can be positioned and spaced according to the intended application. For example, a wound covering might include barbs located at the periphery of a blood plasma-derived scaffold, as shown in Figure 5A. Other non-limiting embodiments include the use of barbs of differing lengths in the same apparatus, or positioning of the barbs in different arrangements. Figure 5B, for example, shows barbs positioned at one edge of a scaffold, allowing for the scaffold to be wrapped around a tendon, ligament, bone, or other injured tissue, wherein the barbs attach to the scaffold material to maintain the wrap or tube-like shape of the tissue or scaffold and avoiding the need to suture the material. In some embodiments, the barbs are positioned across one face of the scaffold.

## Claims

1. A blood plasma-derived plastic scaffold comprising one or more barbs or spikes, wherein the barbs or spikes are positioned across one face of the scaffold, along one edge of the scaffold, along the periphery of the scaffold, or any combination thereof, and wherein the barbs or spikes are insert molded onto the scaffold.

2. The scaffold of claim 1, wherein the scaffold comprises one or more barbs and the barbs comprise a biocompatible polymer.

3. The scaffold of claim 2, wherein the polymer is selected from the group consisting of a polymethyl methacrylate and a bioresorbable polylactic acid.

4. The scaffold of claim 1, wherein the scaffold comprises one or more barbs and the barbs comprise a blood plasma-derived plastic.

5. The scaffold of claim 4, wherein the blood plasma-derived plastic is a different formulation than that of the scaffold.

6. The scaffold of claim 1, wherein the scaffold comprises stacked or laminated layers of blood-derived plastic sheets.

7. The blood plasma-derived plastic scaffold comprising one or more barbs or spikes according to claim 1 for use in treating a wound, wherein the wound is to be contacted with the scaffold.

8. The scaffold for the use of claim 7, wherein the wound is selected from the group consisting of: ulcerated skin, burned skin, tendon injuries, ligament injuries, nerve damage, and muscular injuries.

9. The scaffold for the use of claim 8, wherein the ulcerated skin is a diabetic foot ulcer.

10. A wound covering comprising a blood plasma-derived plastic scaffold and one or more barbs or spikes, wherein the barbs or spikes are positioned across one face of the scaffold, along one edge of the scaffold, along the periphery of the scaffold, or any combination thereof, and wherein the barbs or spikes are insert molded onto the scaffold.

11. The wound covering of claim 10, wherein the barbs or spikes are positioned across one face of the scaffold or the barbs or spikes are positioned along the periphery of the scaffold.

12. A wrap comprising a blood plasma-derived plastic scaffold and one or more barbs or spikes, wherein the barbs or spikes are positioned along one edge of the scaffold and wherein the barbs or spikes are inserted molded on to the scaffold.

## Patentansprüche

1. Ein aus Blutplasma abgeleiteter Plastik-Scaffold, umfassend eine oder mehrere Spitzen oder Stacheln, wobei die Spitzen oder Stacheln über eine Fläche des Scaffolds, entlang einer Kante des Scaffolds, entlang des Umfangs des Scaffolds oder jeglicher Kombination davon positioniert sind und wobei die Spitzen oder Stacheln auf den Scaffold umspritzt sind.

2. Der Scaffold von Anspruch 1, wobei der Scaffold eine oder mehrere Spitzen umfasst und die Spitzen ein biokompatibles Polymer umfassen.

3. Der Scaffold von Anspruch 2, wobei das Polymer ausgewählt ist aus der Gruppe bestehend aus einem Polymethylmethacrylat und einem bioresorbierbaren Polylactid.

4. Der Scaffold von Anspruch 1, wobei der Scaffold eine oder mehrere Spitzen umfasst und die Spitzen ein aus Blutplasma abgeleitetes Plastik umfassen.

5. Der Scaffold von Anspruch 4, wobei das aus Blutplasma abgeleitete Plastik eine andere Zusammensetzung als jene des Scaffolds ist.

6. Der Scaffold von Anspruch 1, wobei der Scaffold geschichtete oder laminierte Schichten von aus Blut abgeleiteten Plastik-Lagen umfasst.

7. Der aus Blutplasma abgeleitete Plastik-Scaffold, umfassend eine oder mehrere Spitzen oder Stacheln gemäß Anspruch 1 zur Verwendung bei der Behandlung einer Wunde, wobei die Wunde mit dem Scaffold in Kontakt zu bringen ist.

8. Der Scaffold zur Verwendung von Anspruch 7, wobei die Wunde ausgewählt ist aus der Gruppe bestehend aus: ulzerierter Haut, verbrannter Haut, Sehnenverletzungen, Bandverletzungen, Nervenschaden und muskulären Verletzungen.

9. Der Scaffold zur Verwendung von Anspruch 8, wobei die ulzerierte Haut ein diabetisches Fuß-Ulcus ist.

10. Eine Wundabdeckung, umfassend einen aus Blutplasma abgeleiteten Plastik-Scaffold und eine oder mehrere Spitzen oder Stacheln, wobei die Spitzen oder Stacheln über eine Fläche des Scaffolds, entlang einer Kante des Scaffolds, entlang des Umfangs des Scaffolds oder jeglicher Kombination davon positioniert sind und wobei die Spitzen oder Stacheln auf den Scaffold umspritzt sind.

11. Die Wundabdeckung von Anspruch 10, wobei die Spitzen oder Stacheln über eine Fläche des Scaffolds positioniert sind oder die Spitzen und Stacheln entlang des Umfangs des Scaffolds positioniert sind.

12. Eine Umwicklung, umfassend einen aus Blutplasma abgeleiteten Plastik-Scaffold und eine oder mehrere Spitzen oder Stacheln, wobei die Spitzen oder Stacheln entlang einer Kante des Scaffolds positioniert sind und wobei die Spitzen oder Stacheln auf den Scaffold umspritzt sind.

## Revendications

1. Échafaudage en plastique dérivé de plasma sanguin comprenant un ou plusieurs ardillons ou pointes, dans lequel les ardillons ou pointes sont positionné(e)s sur une face de l'échafaudage, le long d'un bord de l'échafaudage, le long de la périphérie de l'échafaudage, ou une quelconque combinaison de ceux-ci, et dans lequel les ardillons ou pointes sont inséré(e)s moulé(e)s sur l'échafaudage.

2. Échafaudage selon la revendication 1, dans lequel l'échafaudage comprend un ou plusieurs ardillons et les ardillons comprennent un polymère biocompatible.

3. Échafaudage selon la revendication 2, dans lequel le polymère est choisi dans le groupe consistant en un polyméthacrylate de méthyle et un acide polylactique biorésorbable.

4. Échafaudage selon la revendication 1, dans lequel l'échafaudage comprend un ou plusieurs ardillons et les ardillons comprennent une matière plastique dérivée de plasma sanguin.

5. Échafaudage selon la revendication 4, dans lequel la matière plastique dérivée de plasma sanguin est une formulation différente de celle de l'échafaudage.

6. Échafaudage selon la revendication 1, dans lequel l'échafaudage comprend des couches empilées ou stratifiées de feuilles de plastique dérivées du sang.

7. Échafaudage en plastique dérivé de plasma sanguin comprenant un ou plusieurs ardillons ou pointes selon la revendication 1, pour une utilisation dans le traitement d'une plaie, dans lequel la plaie doit être mise en contact avec l'échafaudage.

8. Echafaudage pour l'utilisation selon la revendication 7, dans lequel la plaie est choisie dans le groupe consistant en peau ulcérée, peau brûlée, lésions des tendons, lésions des ligaments, dommage des nerfs et lésions musculaires.

9. Echafaudage pour l'utilisation selon la revendication 8, dans lequel la peau ulcérée est un ulcère du pied diabétique.

10. Recouvrement d'une plaie comprenant un échafaudage en plastique dérivé du plasma sanguin et un ou plusieurs ardillons ou pointes, dans lequel les ardillons ou pointes sont positionné(e)s sur une face de l'échafaudage, le long d'un bord de l'échafaudage, le long de la périphérie de l'échafaudage ou une quelconque combinaison de ceux-ci, et dans lequel les ardillons ou pointes sont inséré(e)s moulé(e)s sur l'échafaudage.

11. Recouvrement d'une plaie selon la revendication 10, dans lequel les ardillons ou pointes sont positionné(e)s sur une face de l'échafaudage ou les ardillons ou pointes sont positionné(e)s le long de la périphérie de l'échafaudage.

12. Enveloppement comprenant un échafaudage en plastique dérivé de plasma sanguin et un ou plusieurs ardillons ou pointes, dans lequel les ardillons ou pointes sont positionné(e)s le long d'un bord de l'échafaudage et dans lequel les ardillons ou pointes sont inséré(e)s moulé(e)s sur l'échafaudage.
